# EUROPEAN PATENT SPECIFICATION

(11) **EP 3 849 995 B1**
(45) Date of publication and mention of the grant of the patent: **09.11.2022**
(21) Application number: 19773750.5
(22) Date of filing: 11.09.2019
(51) Int. Cl.: C07K 1/32, C07D 487/22

(54) **METHODS FOR SEPARATION OF CHLOROPHYLL AND SOLUBLE PROTEINS**
VERFAHREN ZUR TRENNUNG VON CHLOROPHYLL UND LÖSLICHEN PROTEINEN
PROCÉDÉS DE SÉPARATION DE CHLOROPHYLLE ET DE PROTÉINES SOLUBLES

(30) Priority: 11.09.2018 DK PA201870581
(43) Date of publication of application: 21.07.2021
(73) Proprietor: Lihme Protein Solutions APS, 3520 Farum (DK)
(72) Inventor: LIHME, Allan, Otto, Fog, 3520 Farum (DK); LINDVED, Bodil, Kjær, 3060 Espergærde (DK); HANSEN, Marie, Bendix, 2000 Frederiksberg (DK)
(74) Representative: Inspicos P/S
(86) International application number: PCT/EP2019/074220
(87) International publication number: WO 2020/053272

(56) References cited:
- WO-A2-2018/162557
- CN-A- 1 087 893
- CN-A- 1 477 197
- CN-A- 101 318 963
- CN-A- 101 985 373
- CN-A- 105 646 501
- CN-A- 106 430 485

## Description

### FIELD OF THE INVENTION

The present invention relates to methods for separation of chlorophyll and soluble proteins, such as rubisco, using silicates.

### BACKGROUND OF THE INVENTION

The present invention relates to a method for separation of chlorophyll and soluble proteins, such as rubisco, using soluble silicates, and chlorophyll and protein products produced using such methods.

Chlorophyll is present in high concentrations in the cellular organelles that allows for organisms to produce their own energy through photosynthesis. All plants and several different types of microorganisms go through photosynthesis. Algae is a broad term that includes several different types of photosynthetic microorganisms, and there are several different types of chlorophyll present in algae.

Chlorophyll a is found in all organisms that photosynthesize, including algae and photosynthesizing bacteria. Chlorophyll a is a green pigment, which is what gives plants and many algae their natural green color.

Chlorophyll b is a green chlorophyll pigment found in plants and green algae. Chlorophyll b augments chlorophyll a's ability to capture sunlight. Green algae is a broad, informal classification of organisms that includes both Kingdom Monera (single-celled organisms that do not have a nucleus) and Kingdom Protista (more complex single-celled organisms that do have a nucleus). Green algae are the most common organisms found in fresh water and the ocean, and they are a major supplier of oxygen, which is produced during photosynthesis. Chlorophyll c occurs in certain types of algae, including dinoflagellates. Chlorophyll c is a reddish-brown pigment and gives dinoflagellates their distinctive color.

Chlorophyll is an essential compound in many everyday products. It is used not only as an additive in pharmaceutical and cosmetic products but also as a natural food colouring agent. Additionally, it has been reported to have antioxidant and antimutagenic properties.

In green plants, the majority of chlorophylls are attached by non-covalent bonds to protein existing in the form of chlorophyll-protein complexes, including photosystem I (PS I), photosystem II (PS II), and Cytb6/f complexes. All of chlorophyll-protein complexes are located in the electron transport chain of the thylakoid membrane.

Leaf and grass proteins are potentially the cheapest and most abundant source of protein in the world. They are also highly nutritious and have many desirable functional characteristics which could make them useful in both food and industrial products. It is well known that soluble leaf proteins are found in all known chlorophyll-containing plants. The present invention pertains to soluble leaf and grass proteins. Approximately half of the soluble protein in plant leaves is made up of "rubisco" (ribulose-l,5-bisphosphate carboxylase/oxygenase or "RuBisCO").

In C3 plants RuBisCO molecules are found densely packed within chloroplast stroma at concentrations up to 300 mg/mL. The oligomeric protein (MW 550,000) is composed of eight large and eight small subunits which combine to form a compact, nearly spherical molecule. Rubisco, which is found in all known green plants, appears to be the most abundant leaf protein, and it may be the most abundant protein on earth. Rubisco is the enzyme which catalyzes both the carboxylation and oxygenation of RUBP in plants, i.e., the key reactions in photosynthesis and photorespiration. Rubisco has nutritional value comparable to casein. Studies have further shown that rubisco has a significantly higher Protein Efficiency Ratio (PER, i.e., weight gained/protein consumed) than either casein or egg protein. Rubisco also has excellent binding, gelling, foaming, whipping and emulsifying characteristics. When highly purified rubisco is furthermore colourless, tasteless and odourless, which makes it attractive for incorporation into food or industrial products. Given these desirable nutritional and functional properties, rubisco may prove suitable for incorporation into a range of both food and non-food products for such purposes as a nutritional supplement, binding agent or emulsifier.

The remaining half of soluble leaf proteins share many of the same beneficial traits as rubisco. They have a PER and nutritional quality comparable with casein.

Leaf proteins have been the target for commercial development and production as nutritional and functional products for human food application for more than half a century. However, the protein extraction methods developed have either resulted in green protein preparations which have an odour, taste and texture rendering them undesirable for human consumption at least in part due to inadequate removal of chlorophylls, or they have not been commercially viable due to high losses of protein and functionality or high cost processing steps have been applied.

Techniques for industrial scale isolation of proteins from complex liquid raw materials have been a target of constant development for more than a century. Very many different methods based on various physico-chemical parameters have been described in the prior art but only few have found industrial applicability.

Precipitation of proteins from aqueous solutions is widely used for large scale separation. Proteins may be precipitated by adding various agents such as organic solvents, lyotropic salts (such as ammonium sulfate) or polymers of different kind. Many food proteins are isolated from plant extracts (such as aqueous extracts of soy beans and peas) by so-called isoelectric precipitation which is based on the natural tendency of some proteins to become insoluble at pH values where the protein surface exhibits a near zero net charge. Isoelectric precipitation of proteins is generally a very low-cost operation. However, the method has limitations due to a rather low selectivity, co-precipitation of other unwanted substances and a narrow window of operation. A major drawback of the isoelectric precipitation method is that it is difficult to remove the co-precipitated impurities by washing of the precipitated proteins because any change of the conditions (such as pH, temperature and ionic strength) may lead to solubilization and loss of the protein. Another major drawback of the isoelectric precipitation method is that only certain proteins will precipitate, leaving significant amounts of otherwise valuable proteins in the mother liquid and thereby lead to economic losses and environmental burdens from the associated waste water. Precipitation of proteins by the addition of chemical substances such as organic solvents, lyotropic salts and polymers is not generally applied for the industrial separation of food and feed grade proteins due to the high costs associated with the chemicals, the high costs of chemicals recycling and treatment of waste water and the need to completely remove these chemicals from the product after the precipitation process.

Precipitation of proteins from aqueous solutions may also be performed by the application of heat, such as heating to 110-130 degrees Celsius under increased pressure, or by heating combined with adjustment of pH to highly acidic pH values. Such processes are industrially applied, for example in order to precipitate potato proteins from potato fruit juice produced as a side-stream in the potato starch manufacturing industry. Such processes may be highly efficient; however, the proteins will be completely denatured by the process conditions. Typically, such treated proteins will be largely insoluble and any biological activity and functional characteristics will be lost. The separation of chlorophyll from plant proteins have also been attempted by the application of heat treatment, however, even at the lower temperatures efficient for the separation a significant yield loss of proteins appears.

Membrane filtration is another widely and industrially used method for the isolation and concentration of proteins from complex mixtures. The fundamental separation principle is based on the passing of the liquid through semi-permeable membranes allowing only the passage of molecules smaller than the size of the porous structure of the membrane. Thus, membrane filtration separates molecules largely on the basis of their size and the availability of membranes with different pore sizes enables the separation of molecules and particles of varying size ranges. However, to achieve an efficient separation, the molecules to be separated must have very different sizes (such as at least 10 times different size). Molecules being closer in size will only be partially separated which may be detrimental to the product yield and thereby the economy of the separation process.

Solid phase adsorption (adsorption chromatography) is based on the reversible interaction of molecules in a solution with the surface structures of an insoluble adsorbent material. Silica gels, in the form of silicon dioxide beads or coarse granules, constitute a specific type of solid phase adsorbents that may be produced with varying pore size and available surface area. Agarose beads and synthetic polymer beads constitute other groups of solid phase adsorbents with different characteristics for different protein separation tasks. The surface of the insoluble adsorbent material may be chemically derivatized to facilitate interaction with molecules of widely different nature and can be designed to achieve highly selective separation of even closely related molecules. Thus, solid phase adsorption is widely applied in the manufacture of proteins for pharmaceutical applications.

Due to the high selectivity of solid phase adsorption this methodology has attracted much attention for separation tasks requiring high product purity. However, the cost of the adsorbents, the time-consuming cycling between binding and release of target molecules and the high water and chemicals consumption for washing, cleaning and regeneration of the adsorbents all adds to the high cost of using this separation technology. Therefore, solid phase separation is only rarely used for the isolation of food and feed grade proteins.

Most of the currently applied protein isolation methods are negatively influenced by the presence of chlorophyll pigments in the raw material. The heterogeneous nature, with a high content of large colloid aggregates, of chlorophyll associated pigments tends to make it very difficult to separate the pigments and the proteins in simple and high yielding steps.

From another perspective separated chlorophyll containing fractions may have a rich nutritional profile (e.g. insoluble proteins, dietary fibres, minerals and secondary metabolites) and consist of complex biological structures (e.g. chloroplastic membranes) that may be used as raw materials for valuable products if mild processing steps, avoiding denaturation and break down, are applied. The documents CN105646501, CN1087893 or CN101318963 disclose processes for the extraction of chlorophyll and proteins from biological material. Organic solvents are used in these processes.

Accordingly, there is a need for methods for separating chlorophyll and proteins in aqueous solutions as well as methods for isolation of valuable products from the separated fractions.

### SUMMARY

In a first aspect, the present invention relates to a method for separating soluble proteins from chlorophyll in an aqueous protein solution comprising said protein and chlorophyll, the method comprising;
a. providing an aqueous solution containing soluble protein and chlorophyll
b. adding a water-soluble silicate to the solution of step a) such that the total concentration of silicon in the form of free or complexed silicates in the solution is in the range of 1-500 mM, such as 2-300 mM, such as 3-200 mM, such as 3.5-100 mM, such as 4-60 mM, such as 4-50 mM, such as 5-30 mM
c. if necessary, adjusting the pH of the resulting solution to a pH in the range of pH 5 to pH 11, such a pH in the range of pH 5.5 to pH 10, such a pH in the range of pH 6.0 to pH 9.5, such a pH in the range of pH 6.2 to pH 9.0, such a pH in the range of pH 6.5 to pH 8.5, such as a pH in the range of pH 6.0 to pH 7.5
d. allowing the silicate to form an insoluble precipitate comprising silicate-chlorophyll complexes, while the soluble protein remains soluble in the solution
e. separating the silicate-chlorophyll complexes from the protein solution as a wet precipitate; such as a wet cake or an aqueous suspension of the precipitate,
f. optionally washing the silicate-chlorophyll complexes,
g. optionally separating the chlorophyll from the silicate,
h. optionally isolating the protein from the protein solution obtained in step e),
thereby obtaining the protein and chlorophyll in separated fractions.

### LEGENDS TO THE FIGURES

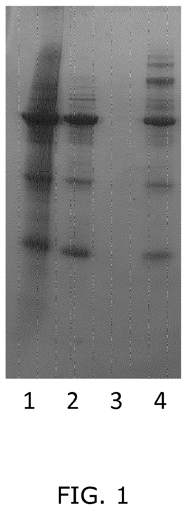

Figures 1-2 show SDS-PAGE analyses of the various solutions of examples 1-3.

### DETAILED DISCLOSURE OF THE INVENTION

The term "chlorophyll" means any of several related green pigments in free or complexed form found in cyanobacteria and the chloroplasts of algae and plants.

The term "anionic compound" means a compound that comprise a negatively charged moiety at a pH in the range of pH 3 to pH 13.

The term "dry weight" means the weight or mass of a substance remaining after removal of water by heating to constant weight at 110 degrees Celcius. The dry weight per ml sample is thus the weight or mass of a substance remaining after removal of water by heating to constant weight at 110 degrees Celcius per ml sample applied to drying.

The term "isolating" or "separating" means any human intervention which change the relative amount of the compound compared to another selected constituent in a given matrix to a higher relative amount of the compound relative to the other constituent. In an embodiment, the compound may be isolated into a pure or substantially pure form. In this context, a substantially pure compound means that the compound preparation contains less than 10%, such as less than 8%, such as less than 6%, such as less than 5%, such as less than 4%, such as less than 3%, such as less than 2%, such as less than 1 %, such as less than 0.5% by weight of other selected constituents. In an embodiment, an isolated compound is at least 50% pure, such as at least 60% pure, such as at least 80% pure, such as at least 90% pure, such as at least 91% pure, such as at least 92% pure, such as at least 93% pure, such as at least 94% pure, such as at least 95% pure, such as at least 96% pure, such as at least 97% pure, such as at least 98% pure, such as at least 99% pure, such as at least 99.5% pure, such as 100 % pure by dry weight.

The term "membrane separation process" refers to a process using a semi-permeable membrane, allowing only compounds having a size lower that a certain value to pass, to separate molecules of a higher size in a liquid or gas continuous phase composition from molecules of a lower size. In this context, liquid or gas continuous phase compositions are to be understood in the broadest sense, including both single phase compositions such as solutions or gases, and dual phase compositions such as slurries, suspensions or dispersions wherein a solid is distributed in a liquid or gas phase.

The term "retentate" means compounds which are not allowed to pass a selected membrane in a membrane separation process.

The term "permeate" or "filtrate" means compounds which can pass a selected membrane in a membrane separation process.

The term "precipitation" refers to the phenomenon that a dissolved compound exceeding its solubility in the solvent undergoes a phase transition from a dissolved liquid state to a solid state. Precipitation is often caused by a chemical reaction and/or a change in the solution conditions. The solidified compound is referred to as the "precipitate".

The term "diafiltration" means a technique that uses ultrafiltration membranes to completely remove, replace, or lower the concentration of salts or solvents from solutions containing proteins, peptides, nucleic acids, and other biomolecules. The process selectively utilizes permeable (porous) membrane filters to separate the components of solutions and suspensions based on their molecular size. An ultrafiltration membrane retains molecules that are larger than the pores of the membrane while smaller molecules such as salts, solvents and water, which are 100% permeable, freely pass through the membrane. In a diafiltration process the retentate is added water or a buffer composition while the membrane filtration process continuously removes water, salts and low molecular weight compounds to the permeate side of the membrane.

The term "adsorption" means a process in which molecules from a gas, liquid or dissolved solid adhere to a surface of a solid phase adsorbent. Likewise, and adsorbent (also named a solid phase adsorbent) is an insoluble material on which adsorption can occur.

The term "protein concentration" means the amount of protein per liter of a sample calculated as the total weight or mass of amino acids per liter as determined according to EUROPEAN PHARMACOPOEIA 5.0 section 2.2.56. AMINO ACID ANALYSIS or by determination of total nitrogen in a sample by the method of Kjeldahl using the conversion factor N x 6,25. All samples are dialyzed against demineralized water in dialysis tubing cellulose membrane (Sigma-Aldrich, USA, cat. No.: D9652) to remove any free amino acids and low molecular weight peptides prior to the amino acid determination.

The term "protein purity" means the relative amount of protein of a dried sample wherein the total weight or mass of amino acids per gram is determined according to EUROPEAN PHARMACOPOEIA 5.0 section 2.2.56. AMINO ACID ANALYSIS or by determination of total nitrogen in a sample by the method of Kjeldahl using the conversion factor N x 6,25 %. All samples are, prior to drying, dialyzed against demineralized water in dialysis tubing cellulose membrane (Sigma-Aldrich, USA, cat. No.: D9652) to remove any free amino acids and low molecular weight peptides prior to the amino acid or nitrogen determination. "Protein purity" is indicated in percent (gram protein/gram x 100 %).

The term "soluble" means solubility in water at a concentration of at least 1 g/L at 25 degrees Celsius.

The term "comprise" and "include" as used throughout the specification and the accompanying items/claims as well as variations such as "comprises", "comprising", "includes" and "including" are to be interpreted inclusively. These words are intended to convey the possible inclusion of other elements or integers not specifically recited, where the context allows.

The articles "a" and "an" are used herein to refer to one or to more than one (i.e. to one or at least one) of the grammatical object of the article. By way of example, "an element" may mean one element or more than one element.

The key findings of the present technology are that an aqueous solution containing soluble protein and chlorophyll may be separated into two individual fractions; one fraction significantly enriched in soluble protein relative to chlorophyll and another fraction significantly enriched in chlorophyll relative to protein when compared to the initial aqueous solution, by the addition of a water soluble silicate, and optionally adjusting pH, to achieve a selective precipitation of insoluble silicate-chlorophyll complexes. Another key finding of the present technology is that the two fractions thus obtained are excellent raw materials for further processing to achieve highly purified protein and chlorophyll containing products for human food, animal feed, enzymatic reactions, cosmetics, healthcare applications and fermentation purposes.

The separation methods applied according to the invention are very mild and thus retains the solubility and bioactivity of fragile proteins including the enzymatic activity of enzymes present in the raw material. This contrasts with prior art methods using more harsh treatments, such as the application of temperatures above 50 °C, which leads to pronounced denaturation and loss of protein yields.

Thus, there is a need for more efficient and mild separation methods for the provision of commercially viable manufacturing processes.

A method for separating soluble proteins from chlorophyll in an aqueous protein solution comprising said protein and chlorophyll is thus provided, the method comprising;
a. providing an aqueous solution containing soluble protein and chlorophyll
b. adding a water-soluble silicate to the solution of step a) such that the total concentration of silicon in the form of free or complexed silicates in the solution is in the range of 1-500 mM, such as 2-300 mM, such as 3-200 mM, such as 3.5-100 mM, such as 4-60 mM, such as 4-50 mM, such as 5-30 mM
c. if necessary, adjusting the pH of the resulting solution to a pH in the range of pH 5 to pH 11, such a pH in the range of pH 5.5 to pH 10, such a pH in the range of pH 6.0 to pH 9.5, such a pH in the range of pH 6.2 to pH 9.0, such a pH in the range of pH 6.5 to pH 8.5, such as a pH in the range of pH 6.0 to pH 7.5
d. allowing the silicate to form an insoluble precipitate comprising silicate-chlorophyll complexes, while the soluble protein remains soluble in the solution
e. separating the silicate-chlorophyll complexes from the protein solution as a wet precipitate; such as a wet cake or an aqueous suspension of the precipitate,
f. optionally washing the silicate-chlorophyll complexes,
g. optionally separating the chlorophyll from the silicate,
h. optionally isolating the protein from the protein solution obtained in step e),
thereby obtaining the protein and chlorophyll in separated fractions.

In one aspect, said washing step f) is mandatory. In another aspect, said separating step g) is mandatory. In yet another aspect, said separation step h) is mandatory. Said method may further comprise a step of filtration to remove insoluble particles or fibres prior to step b).

### Silicates

A silicate in the context of the present invention is an anionic compound containing silicon. Any water-soluble silicate may be employed according to the invention. Particularly preferred are the alkali metal silicates including sodium ortho silicates comprising the anion SiO₄ ⁴⁻ . Also, known as water glass or liquid glass, these materials are available in aqueous solution and in solid form.

The silicate concentration is in the range of 0.5-50 g/L in the present context may preferably be in the range of 0.5-25 g/L, 0.5-17 g/L, 1-15 g/L, 1-12 g/L, 1-10 g/L, 1-8 g/L, 1.5-20 g/L, 1.5-15 g/L, 1.5-12 g/L, 2-20 g/L, 2-15 g/L, 2-12 g/L, 2.5-20 g/L, 2.5-15 g/L, or 2.5-12 g/L. The silicate concentration may be in the range of 1-6 g/L, preferably in the range of 1.5-4 g/L.

In some embodiments the total concentration of silicon in the form of free or complexed silicates in the solution is in the range of 1-500 mM, such as 2-300 mM, such as 3-200 mM, such as 3- 100 mM, such as 3-30 mM, such as 3- 20 mM, such as 3.5-100 mM, such as 4-60 mM, such as 4-50 mM, such as 5-30 mM.

### Sources of an aqueous protein solution comprising protein and chlorophyll.

Preferred raw materials for the aqueous protein solutions comprising protein and chlorophyll according to the invention are plant leaves, stems and pods; cyanobacteria, algae and aquatic plants.

In a preferred embodiment the plant leaves, pods and stems originate from agricultural crops such as grasses, alfalfa, potato, sweet potato, spinach, sorghum, cassava, rice, sugar beets, sugar cane, tobacco, beans and peas.

Spirulina is filamentous, helical, photosynthetic cyanobacteria naturally inhabiting alkaline brackish and saline waters in tropical and subtropical regions. Biochemical analysis has revealed its exceptional nutritive properties, so it is referred in the literature as "super food" or "food of the future". Spirulina is one of the richest natural sources of proteins and essential amino acids, as well as an excellent source of vitamins (primarily A, K, and vitamin B complex), macro- and micro-elements (calcium, potassium, magnesium, iron, iodine, selenium, chromium, zinc, and manganese), essential fatty acids, including γ-linoleic acid (GLA), glycolipids, lipopolysaccharides, and sulfolipids. Spirulina is especially rich in a variety of pigments, such as chlorophylls, β-carotene, xanthophylls, and phycobilins (phycobiliproteins).

In a preferred embodiment a raw material for the aqueous protein solution according to the invention is a cyanobacterium, preferably *Arthrospira platensis* and/or *Arthrospira maxima.*

Aquatic plants represent a further preferred source of raw materials for the aqueous protein solutions of the invention. Duckweed is an aquatic plant of the Lemna family and is particularly rich in proteins. Duckweed is small green freshwater plants with fronds from 1 to 12 mm in diameter. They are the smallest and simplest flowering plants and have one of the fastest production rates with a doubling time of 2 to 3 days only. This is because all the plant consists of metabolically active cells with very little structural fiber. Some of the specific properties of duckweed are that the plants have the capability of converting degradable pollutants directly into protein rich fodder.

In a preferred embodiment a raw material for the aqueous protein solution according to the invention is an aquatic plant, such as duckweed.

The aqueous protein solution comprising said protein and chlorophyll are typically produced by disintegration, e.g. by grinding, shredding and/or pressing, of the raw materials whereby an aqueous solution comprising protein and chlorophyll is released as a juice, and/or the components may be extracted by addition of water or an aqueous extractant solution in combination with physical disruption of the plant tissue and cells.

The temperature of operating the different steps according to the invention may be the same or different temperatures. Preferred embodiments comprise the use of temperatures that are generally non-denaturing to proteins. In a preferred embodiment the temperature of said step d) is in the range of 5-55 °C, such as 7-50 °C, such as 10-48 °C, such as 15-45 °C, such as 15-40 °C, such as 10-30 °C.

The choice of operating parameters such as pH, temperature and silicate concentration in said steps b) through f) influence how much of said soluble protein will remain in solution and how much will co-precipitate with the chlorophyll-silicate complexes. Generally speaking the higher the silicate concentration, the more soluble protein may co-precipitate with the chlorophyll. In a preferred embodiment the insoluble precipitate of said step d) contains less than 50 %, such as less than 40 %, such as less than 30 %, such as less than 25 %, such as less than 20 %, such as less than 15 %, such as less than 10 % of said soluble protein.

In a preferred embodiment we claim a chlorophyll-silicate product produced according to the invention. In a preferred embodiment we claim a chlorophyll-silicate product comprising 10-99 %, such as 15-95%, 20-90 %, 30-90 %, 35-90 %, 40-90% of chlorophyll, and 1-90%, such as 5-85%, such as 10-80%, such as 10-70, such as 10-65%, such as 10-60% of silicate, on a dry weight basis.

The isolated chlorophyll-silicate complexes and the isolated chlorophyll may be used in a number of highly valuable applications and in preferred embodiments the invention comprise such chlorophyll and chlorophyll-silicate complexes. The use of said chlorophyll or chlorophyll-silicate products will in preferred embodiments be as a raw material or an ingredient for a food, a feed, a cosmetic, a dietary supplement or a healthcare product.

In a preferred embodiment the use of a chlorophyll-silicate product according to the invention is as a raw material or an ingredient for a satiety and/or a weight controlling product.

In a preferred embodiment the use of a chlorophyll-silicate product according to the invention is as a raw material or an ingredient for a fish feed product. In a preferred embodiment the use of a chlorophyll-silicate product according to the invention is as a raw material or an ingredient as a nutrient in fermentation of microorganisms. In a preferred embodiment the use of a chlorophyll-silicate product according to the invention is as a raw material or an ingredient as a nutrient in a fertilizer.

In order to enhance the usefulness of such chlorophyll-silicate products certain impurities may need to be fully or partially removed. Therefore, in a preferred embodiment the isolated chlorophyll-silicate complexes are extracted with one or more of organic solvents, acid, base, detergents or high ionic strength aqueous solutions or combinations of these to separate one or more of phenols, pigments, phytates, saponins, tannins or protease inhibitors therefrom.

In a preferred embodiment the protein from the protein solution obtained in step e) is isolated by a method comprising further treatment of the protein solution using tangential flow membrane filtration wherein the protein is retained in the retentate and impurities are passing the membrane as a permeate

In a preferred embodiment the protein from the protein solution obtained in step e) is isolated by a method comprising acidification of the protein solution to form an insoluble precipitate of the protein and isolating the precipitate. In a preferred embodiment the acidification is obtained by fermentation, preferably by lactic acid fermentation.

In a preferred embodiment the protein from the protein solution obtained in step e) is isolated by a method comprising further silicate addition to the protein solution and, if necessary adjustment of pH, to form an insoluble precipitate of protein-silicate complexes and isolating the precipitate.

In a preferred embodiment the protein-silicate is further processed to separate the protein from the silicate.

The purity of the isolated protein may determine its value of use in various applications while the yield and the cost of processing as well may be influenced by the process parameters applied to reach a certain purity. For some applications the protein purity may not need to be very high as long as the cost of manufacture is kept as low as possible. Thus is a preferred embodiment the protein from the protein solution obtained in step e) is isolated to reach a purity of at least 50 %, such as at least 60 %, such as at least 65 %, such as at least 70 %, such as at least 75 % such as at least 80 %, such as at least 85 %, such as at least 90 %, such as at least 92 % as determined by the Kjeldahl method (N x 6.25 %) on dried protein samples.

### EXAMPLES

### Materials and methods

Chemicals used in the examples herein e.g. for preparing buffers and solutions are commercial products of at least reagent grade.

Waterglass, sodium silicate used for precipitation of proteins was from Borup Kemi, Denmark, 36°BE, d = 1.33 g/ml, SiO₂ = 25-26 w/w % and Na₂0 = 7.5-8.5 w/w %.

Water used for conducting the experiments is all de-ionized water

### Buffer solutions

A 10 wt% sodium sulphite buffer solution is prepared by dissolving 10 g of sodium sulphite from Sigma Aldrich USA (cat. No.: 13471) in 100 mL water. pH was not adjusted. Measured to pH 7.7.

A 50 mM sodium chloride solution is prepared by dissolving 2.9 g of sodium chloride in 1 L of water.

### SDS-PAGE electrophoresis reagents

a) LDS sample buffer, 4X is obtained from Expedeon, USA (Cat.no.: NXB31010)
b) SDS Run buffer, 20x is obtained from Expedeon, USA (Cat.no.: NXB50500)
c) Precast 4-20% gradient gels are obtained from Expedeon, USA (Cat.no.: NXG42012K)
d) Instant Blue Coomassie staining solution is obtained from Expedeon, USA (Cat.no.ISB1L).

### Ultrafiltration

Samples are ultrafiltrated using a system from Spectrum Labs, USA, fitted with KrosFlo TFF system KMOi using hollow fiber ultrafiltration membranes. A membrane cut-off value of 10 kDa and a membrane area of 490 cm2 is employed (Spectrum Labs, USA cat.no.: S02-E010-10-N).

### Spinach extract:

Fresh spinach leaves are obtained from a local supermarket.

The fresh spinach leaves (250 g) are washed with water and then blended with an equal amount of water containing 0.14 M NaCl and 0.8 % Na₂SO₃ (sodium sulphite) in a commercial blender. After blending for 5 minutes pH of the homogenate is adjusted to 8.7 with 5 M NaOH followed by further 5 minutes blending. The homogenate is sieved through cheese cloth to remove the larger fibres.

250 g spinach leaves yields about 250-300 ml spinach extract with a pH of 8,5-8,6 and a conductivity of 19 mS/cm, measured with a Seven2Go S3 conductivity meter from Mettler Toledo, Switzerland.

### Sugar beet tops extract:

Sugar beet leaves (tops) were collected fresh from the field and immediately soaked in water containing 1 % sodium sulphite.

To produce sugar beet top extract 1 kg of leaves were pressed through a slow juicer (Angel Slow Juicer). The resulting juice was added an equal amount of water containing 0.4 % sodium sulphite. The dark green juice had a pH of 6.5 and a conductivity of 19.5 mS/cm.

All tests were performed at room temperature (20-25 degrees Celsius unless otherwise indicated.

### Assays

### SDS-PAGE electrophoresis

The samples produced in each example are analyzed using SDS-PAGE gel electrophoresis showing the protein composition in each sample. The SDS-PAGE gel electrophoresis is performed using an electrophoresis apparatus and precast 16% gradient gels from Expedeon USA (Cat.no.: NXG42012K). The protein samples are mixed with LDS sample buffer and incubated for 10 minutes at 70°C. The samples are applied to a precast gel and proteins are allowed run for 70 minutes at 200 V 90 mA in the SDS Run buffer at non-reduced running conditions. The gel is developed in the staining solution for three hours and the protein bands are evaluated by visually inspection.

### Example 1. Isolating protein from spinach extract by precipitation of chlorophyll (step 1) followed by silicate precipitation of protein (step 2)

### Step 1:

200 ml of spinach extract (test solution 1), produced according to materials and methods, is mixed with 1.0 ml of a concentrated solution of sodium silicate (technical grade water glass from Borup Kemi, Denmark, 36-38 degrees Baumé). Addition of the waterglass is performed dropwise. When the full amount of water glass has been added, pH is adjusted to pH 9 with 10% H₂SO₄ and the suspension is standing for 30 minutes at room temperature before adjusting the pH to pH 7.8 with 10% H₂SO₄. The suspension is standing for another 30 minutes while chlorophyll and the silicate forms insoluble complexes and precipitates. The suspension is then centrifuged at 1430 G for 10 minutes, followed by decantation of the supernatant (test solution 2) as a clear (OD 620 nm = 0.05) slightly brown liquid. The precipitated chlorophyll-silicate complexes were retrieved as a dark-green and moist cake (27 gram).

### Step 2:

The supernatant (test solution 2) is mixed with another 2.0 ml of the concentrated solution of sodium silicate (water glass). Addition of the waterglass is performed dropwise. After 30 minutes the suspension is adjusted to pH 6.0 with 10% H₂SO₄ and is standing for another 30 minutes before centrifugation at 1430 G for 10 minutes. The protein depleted supernatant (test solution 3) is discarded.

The precipitate is washed with water 3 times (centrifugation at 1430 G for 30 minutes) and the protein is released from the precipitate by suspension of the precipitate in water and adjustment of pH in the suspension to pH 10.4 by dropwise addition of 1 M sodium hydroxide. After 30 minutes mixing the suspension is centrifuged at 4000 rpm for 5 min and the supernatant is collected to form test solution 4.

SDS-PAGE is performed on test solutions 1 to 4 as illustrated in figure 1.

### RESULTS:

The spinach extract (test solution 1) was a dark-green very unclear solution while the supernatant after chlorophyll precipitation (test solution 2) was a very clear liquid (OD 620 nm = 0.05) with a light brown colour. Thus, practically all the chlorophyll pigments were precipitated and removed from the solution. The precipitated chlorophyll was a high solids moist cake of dark green matter.

### Figure 1: SDS-PAGE of test solutions 1 to 4

Lane 1: Spinach extract (test solution 1)
Lane 2: Supernatant after chlorophyll precipitation (test solution 2)
Lane 3: Supernatant after protein precipitation (test solution 3)
Lane 4: Eluted protein (test solution 4)

Step 1: From the SDS-PAGE of figure 1 it is observed that the major part of the protein in the spinach extract is retained in the supernatant after precipitation of the green chlorophyll with water glass at pH 7.8 (lane 2).

In a control experiment with no added sodium silicate in step 1 the corresponding control test solution 2 was still dark green and unclear after centrifugation.

Step 2: As seen in the SDS-PAGE of figure 1 further addition of water glass and adjustment of pH to pH 6.0 precipitates the protein leaving no detectable protein in the supernatant (lane 3). The subsequently released protein is in the supernatant after adjustment of pH to pH 10.4 (test solution 4). This protein solution was clear and colourless.

The yield of protein in test solution 4 corresponded to approx. 65 % of the protein present in the crude extract (test solution 1) and the purity of the protein in test solution 4 following dialysis against water and drying was 93%

### Example 2. Isolating protein from spinach extract by precipitation of chlorophyll followed by isoelectric precipitation of protein

### Step 1:

200 ml of spinach extract (test solution 1), produced according to materials and methods, is mixed with 1.0 ml of a concentrated solution of sodium metasilicate, technical grade water glass (Borup Kemi, Denmark) 36-38 degrees Baumé. Addition of the waterglass is performed dropwise. When the full amount of water glass has been added, pH is adjusted to pH 9 with 10% H₂SO₄ and the suspension is standing for 30 minutes before lowering the pH to 7.8 with 10% H₂SO₄. The suspension is standing for another 30 minutes while chlorophyll and the silicate forms insoluble complexes and precipitates. The suspension is the centrifuged at 1430 G for 10 minutes.

### Step 2:

The supernatant (test solution 2) is adjusted to pH 4.0 with 10% H₂SO₄ and is incubated for 30 minutes at room temperature to allow the proteins to precipitate before centrifugation at 1430 G for 10 minutes. The supernatant after centrifugation (test solution 3) is decanted and the precipitate is washed three times with water adjusted to pH 4 with 10% H₂SO₄. Following washing the precipitate is dried in a freeze dryer.

### Results:

As in example 1 the spinach extract (test solution 1) was a dark-green very unclear solution while the supernatant after chlorophyll precipitation (test solution 2) was a very clear liquid (OD 620 nm = 0.05) with a light brown colour. The precipitated chlorophyll was a high solids moist cake of dark green matter. SDS-PAGE analysis (not shown) illustrated that the majority of the proteins present in the initial extract was still in solution after removal of the green chlorophyll pigments.

Analysis further showed that by adjustment of pH in the chlorophyll depleted extract (test solution 2) to pH 4.0 all the proteins precipitated efficiently. After washing and drying the precipitated protein had a purity of 90 %.

### Example 3. Isolating protein from spinach extract by precipitation of chlorophyll followed by ultra- and diafiltration.

### Step 1:

2 L of spinach extract (test solution 1), produced according to materials and methods, is mixed with 13 ml of a concentrated solution of sodium metasilicate, technical grade water glass (Borup Kemi, Denmark) 36-38 degrees Baumé. Addition of the waterglass is performed dropwise. When the full amount of water glass has been added, pH is adjusted to pH 9 with 10% H₂SO₄ and the suspension is standing for 30 minutes before lowering the pH to 7.0 with 10% H₂SO₄. The suspension is standing for another 30 minutes while chlorophyll and the silicate forms insoluble complexes and precipitates. The suspension is the centrifuged at 1430 G for 10 minutes. The supernatant (test solution 2) is decanted and the precipitated chlorophyll is washed three times with water and dried in a freeze dryer.

### Step 2:

1.5 L of test solution 2 is subjected to ultrafiltration as described in Materials and Methods using a hollow fiber membrane with a 10 kD cut-off value. When the retentate has reached a volume of 150 ml a volume of 150 ml water is added to the retentate in order to remove low molecular weight compounds by diafiltration. This is repeated 4 times. When the retentate has been diafiltered by addition of 4 times 150 ml water the retentate is collected (test solution 3) and dried.

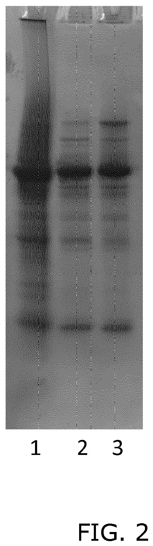

SDS-PAGE is performed on test solutions 1 to 3 as illustrated in figure 2.

### Results:

As in example 1 the spinach extract (test solution 1) was a dark-green very unclear solution while the supernatant after chlorophyll precipitation (test solution 2) was a very clear liquid (OD 620 nm = 0.03) with a light brown colour. The precipitated chlorophyll was a high solids moist cake of dark green matter producing a porous dark powder upon drying. The retentate (test solution 3) following ultrafiltration and diafiltration was a clear and almost colourless solution and the dried protein was an off-white powder.

SDS-PAGE analysis illustrated that the majority of the proteins present in the initial extract was still in solution after removal of the green chlorophyll pigments.

### Figure 2. SDS PAGE analysis of test solution 1-3

Lane 1: Spinach extract (test solution 1)
Lane 2: Supernatant after chlorophyll precipitation (test solution 2)
Lane 3: Retentate after ultrafiltration and diafiltration (test solution 3)

From the SDS-PAGE of figure 2 it is observed that the major part of the protein in the spinach extract is retained in the supernatant after precipitation of the green chlorophyll with water glass at pH 7.0 (lane 2). The protein remained in the retentate during ultrafiltration and diafiltration (lane 3).

The protein purity of the dried test solution 3 was 80 % (N x 6.25 %)

### Example 4. Separating chlorophyll and protein from sugar beet tops extract by precipitation of chlorophyll with sodium silicate.

*Determination of the optimal amount of sodium silicate added for precipitation of chlorophyll.*

Six aliquots of 20 ml of sugar beet top extract (Solutions A, B, C, D, E and F respectively), produced according to materials and methods, are mixed with varying amounts of a concentrated solution of sodium metasilicate, technical grade water glass (Borup Kemi, Denmark) 36-38 degrees Baumé and a content of SiO₂ corresponding to 25-26 w/w %. Addition of the water glass is performed dropwise. When the full amount of water glass has been added, pH is adjusted to pH 9 with 10% H₂SO₄ and the suspension is standing for 30 minutes before lowering the pH to 7.8 with 10% H₂SO₄. The suspension is standing for another 30 minutes while chlorophyll and the silicate forms insoluble complexes and precipitates. The suspension is then centrifuged at 1450 G for 10 minutes. The supernatants are collected to determine the efficiency of chlorophyll precipitation and the protein composition by SDS-PAGE.

The five solutions were added the following amounts of sodium silicate:
A. 0 ml (control)
B. 0.067 ml
C. 0.10 ml
D. 0.13 ml
E. 0.20 ml
F. 0,40 ml

### Results:

The supernatants were examined visually to estimate the remaining chlorophyll content after addition of sodium silicate and centrifugation. Compared to the very dark green control (supernatant A), which had no silicate added, supernatant B and C had still rather high concentration of chlorophyll (more than 50 % and more than 25 % respectively), while supernatant D and E had very low chlorophyll left in solution (less than 10 % for both). Supernatant F had practically no green hue while being very clear and light brown.

When analysing the supernatants by SDS PAGE and performing a semi-quantitative estimate of the protein content left after silicate addition and centrifugation it was found that supernatants A, B and C contained the same level of protein, while supernatants D and E contained approximately 85 % and 80 % of the protein compared to supernatant A. Supernatant F was estimated to contain approx. 50 % of the protein present in the control.

Thus, it was concluded that the optimal amount of sodium silicate to be added to the sugar beet juice would be in the range of 0.13 ml to 0.20 ml per 20 ml juice, corresponding to 6.67 to 10.0 ml sodium silicate per litre sugar beet juice. The sodium silicate solution added contained approx. 25 w/w % SiO₂ equivalents (corresponding to 333 g/L SiO₂ or 5.56 M SiO₂) and thus, the final concentration of silicon in the form of silicates optimally added to the sugar beet juice was found to be in the range of 37 mM to 56 mM. At this concentration and under the conditions of the experiment a near complete separation of chlorophyll from the protein was obtained without losing more than 10-20 % of the protein to the precipitated chlorophyll product.

### Example 5. Separating chlorophyll and protein from various plant juices by precipitation of chlorophyll with sodium silicate.

Extracts/juices from locally sourced and fresh alfalfa, rye grass and common nettle were performed as described for sugar beet leaves in materials and methods.

The obtained dark green juices were added sodium silicate at varying concentrations and further treated as described in example 4 in order to determine the concentration of silicate to be added for optimal separation of chlorophyll and protein in the juices.

For all of the tested juices it was found that the addition of sodium silicate at a concentration in the range of 25 - 50 mM silicon in the form of silicates would result in an efficient chlorophyll precipitation with high yields of protein in the supernatant.

## Claims

1. A method for separating soluble proteins from chlorophyll in an aqueous protein solution comprising said protein and chlorophyll, the method comprising;
a. providing an aqueous solution containing soluble protein and chlorophyll
b. adding a water-soluble silicate to the solution of step a) such that the total concentration of silicon in the form of free or complexed silicates in the solution is in the range of 1-500 mM, such as 2-300 mM, such as 3-200 mM, such as 3- 100 mM, such as 3-30 mM, such as 3- 20 mM, such as 3.5-100 mM, such as 4-60 mM, such as 4-50 mM, such as 5-30 mM
c. if necessary, adjusting the pH of the resulting solution to a pH in the range of pH 5 to pH 11, such a pH in the range of pH 5.5 to pH 10, such a pH in the range of pH 6.0 to pH 9.5, such a pH in the range of pH 6.2 to pH 9.0, such a pH in the range of pH 6.5 to pH 8.5, such as a pH in the range of pH 6.0 to pH 7.5
d. allowing the silicate to form an insoluble precipitate comprising silicate-chlorophyll complexes, while the soluble protein remains soluble in the solution
e. separating the silicate-chlorophyll complexes from the protein solution as a wet precipitate; such as a wet cake or an aqueous suspension of the precipitate,
f. optionally washing the silicate-chlorophyll complexes,
g. optionally separating the chlorophyll from the silicate,
h. optionally isolating the protein from the protein solution obtained in step e),
thereby obtaining the protein and chlorophyll in separated fractions.

2. The method according to claim 1 wherein said washing step f), said separating step g) and/or said isolation step h) is mandatory.

3. The method according to anyone of claims 1-2 further comprising a step of filtration to remove insoluble particles or fibres prior to step b).

4. The method according to any of claims 1-3 wherein the temperature of said step d) is in the range of 5-55 °C, such as 7-50 °C, such as 10-48 °C, such as 15-45 °C, such as 15-40 °C, such as 10-30 °C.

5. The method according to any one of claims 1-4, wherein the insoluble precipitate of step d) contains less than 50 %, such as less than 40 %, such as less than 30 %, such as less than 25 %, such as less than 20 %, such as less than 15 %, such as less than 10 % of said soluble protein.

6. The method according to anyone of claims 1-5 wherein the chlorophyll-silicate complexes separated in said step e) are extracted with one or more of organic solvents, acid, base, detergents or high ionic strength aqueous solutions or combinations of these to separate one or more of phenols, pigments, phytates, saponins, tannins or protease inhibitors therefrom.

7. The method according to any one of the previous claims wherein the protein from the protein solution obtained in step e) is isolated by a method comprising further treatment of the protein solution using tangential flow membrane filtration wherein the protein is retained in the retentate and impurities pass through the membrane as a permeate, or wherein the protein from the protein solution obtained in step e) is isolated by a method comprising acidification of the protein solution to form an insoluble precipitate of the protein and isolating the precipitate, or wherein the protein from the protein solution obtained in step e) is isolated by a method comprising further silicate addition to the protein solution and, if necessary adjustment of pH, to form an insoluble precipitate of protein-silicate complexes and isolating the precipitate.

8. The method according to any one of the previous claims wherein the protein from the protein solution obtained in step e) is isolated to reach a purity of at least 50 %, such as at least 60 %, such as at least 65 %, such as at least 70 %, such as at least 75 %, such as at least 80 %, such as at least 85 %, such as at least, 90 %, such as at least 92 % as determined by the Kjeldahl method (Nx 6.25 %) on dried protein samples.

9. The method according to any one of the previous claims, wherein a raw material for the aqueous protein solution comprising protein and chlorophyll is selected from the group consisting of plant leaves, stems and pods; cyanobacteria, algae and aquatic plants.

10. The method according to claim 9, wherein the plant leaves, pods and stems originate from agricultural crops such as grasses, alfalfa, potato, sweet potato, spinach, sorghum, cassava, rice, sugar beets, sugar cane, tobacco, beans and peas.

11. The method according to claim 9, wherein the cyanobacteria are selected from a Spirulina species, such as *Arthrospira platensis* and/or *Arthrospira maxima.*

12. The method according to claim 9, wherein the aquatic plant is from the Lemna genera, such as duckweed.

13. A chlorophyll-silicate product produced according to any of claims 1-12, preferably comprising 10-99 %, such as 15-95%, 20-90 %, 30-90 %, 35-90 %, 40-90% of chlorophyll, and 1-90%, such as 5-85%, such as 10-80%, such as 10-70, such as 10-65%, such as 10-60% of silicate, on a dry weight basis.

14. The use of a chlorophyll-silicate product according to claim 13 as a raw material or an ingredient for a food, a feed, a cosmetic, a dietary supplement or a healthcare product.

15. The use of a chlorophyll-silicate product according to claim 13 as a raw material or an ingredient for a satiety and/or weight controlling product.

## Patentansprüche

1. Verfahren zum Abtrennen löslicher Proteine von Chlorophyll in einer wässrigen Proteinlösung, die das Protein und Chlorophyll umfasst, wobei das Verfahren Folgendes umfasst:
a. Bereitstellen einer wässrigen Lösung, die lösliches Protein und Chlorophyll enthält
b. Zusetzen eines wasserlöslichen Silicats zu der Lösung aus Schritt a), derart, dass die Gesamtkonzentration von Silicium in Form freier oder komplexierter Silicate in der Lösung im Bereich von 1-500 mM, wie beispielsweise 2-300 mM, wie beispielsweise 3-200 mM, wie beispielsweise 3-100 mM, wie beispielsweise 3-30 mM, wie beispielsweise 3-20 mM, wie beispielsweise 3,5-100 mM, wie beispielsweise 4-60 mM, wie beispielsweise 4-50 mM, wie beispielsweise 5-30 mM liegt
c. Nötigenfalls Einstellen des pH-Werts der resultierenden Lösung auf einen pH-Wert im Bereich von pH 5 bis pH 11, wie beispielsweise einen pH-Wert im Bereich von pH 5,5 bis pH 10, wie beispielsweise einen pH-Wert im Bereich von pH 6,0 bis pH 9,5, wie beispielsweise einen pH-Wert im Bereich von pH 6,2 bis pH 9,0, wie beispielsweise einen pH-Wert im Bereich von pH 6,5 bis pH 8,5, wie beispielsweise einen pH-Wert im Bereich von pH 6,0 bis pH 7,5
d. Gestatten dem Silicat, ein unlösbares Präzipitat zu bilden, das Silicat-Chlorophyll-Komplexe umfasst, während das lösliche Protein in der Lösung löslich bleibt
e. Abtrennen der Silicat-Chlorophyll-Komplexe von der Proteinlösung als nasses Präzipitat; wie beispielsweise einem nassen Kuchen oder einer wässrigen Suspension des Präzipitats,
f. wahlweise Waschen der Silicat-Chlorophyll-Komplexe,
g. wahlweise Abtrennen des Chlorophylls von dem Silicat,
h. wahlweise Isolieren des Proteins von der in Schritt e) erhaltenen Proteinlösung,
wodurch das Protein und Chlorophyll als getrennte Fraktionen erhalten werden.

2. Verfahren nach Anspruch 1, wobei der Waschschritt f), der Abtrennungsschritt g) und/oder der Isolierschritt h) obligatorisch ist/sind.

3. Verfahren nach einem der Ansprüche 1-2, ferner einen Schritt des Filterns zum Entfernen unlöslicher Teilchen oder Fasern vor Schritt b) umfassend.

4. Verfahren nach einem der Ansprüche 1-3, wobei die Temperatur des Schritts d) im Bereich von 5-55 °C, wie beispielsweise 7-50 °C, wie beispielsweise 10-48 °C, wie beispielsweise 15-45 °C, wie beispielsweise 15-40 °C, wie beispielsweise 10-30 °C liegt.

5. Verfahren nach einem der Ansprüche 1-4, wobei das unlösliche Präzipitat aus Schritt d) weniger als 50 %, wie beispielsweise weniger als 40 %, wie beispielsweise weniger als 30 %, wie beispielsweise weniger als 25 %, wie beispielsweise weniger als 20 %, wie beispielsweise weniger als 15 %, wie beispielsweise weniger als 10 % des löslichen Proteins enthält.

6. Verfahren nach einem der Ansprüche 1-5, wobei die in dem Schritt e) abgetrennten Silicat-Chlorophyll-Komplexe mit einem oder mehreren von organischen Lösungsmitteln, Säure, Base, Detergentien oder wässrigen Lösungen hoher Ionenstärke oder Kombinationen dieser extrahiert werden, um eines oder mehrere von Phenolen, Pigmenten, Phytaten, Saponinen, Tanninen oder Proteaseinhibitoren davon abzutrennen.

7. Verfahren nach einem der vorhergehenden Ansprüche, wobei das Protein aus der in Schritt e) erhaltenen Proteinlösung durch ein Verfahren isoliert wird, das die weitere Behandlung der Proteinlösung unter Anwendung von Tangentialflussmembranfiltrierung umfasst, wobei das Protein im Retentat zurückgehalten wird und Verunreinigungen durch die Membran als Permeate hindurchgehen oder wobei das Protein aus der in Schritt e) erhaltenen Proteinlösung durch ein Verfahren isoliert wird, das die Ansäuerung der Proteinlösung zum Bilden eines unlöslichen Präzipitats des Proteins und das Isolieren des Präzipitats umfasst oder wobei das Protein aus der in Schritt e) erhaltenen Proteinlösung durch ein Verfahren isoliert wird, das eine weitere Silicatzugabe zu der Proteinlösung und nötigenfalls das Einstellen des pH-Wert, um ein unlösliches Präzipitat von Protein-Silicat-Komplexen zu bilden, und das Isolieren des Präzipitats umfasst.

8. Verfahren nach einem der vorhergehenden Ansprüche, wobei das Protein von der in Schritt e) erhaltenen Proteinlösung isoliert wird, um eine Reinheit von mindestens 50 %, wie beispielsweise mindestens 60 %, wie beispielsweise mindestens 65 %, wie beispielsweise mindestens 70 %, wie beispielsweise mindestens 75 %, wie beispielsweise mindestens 80 %, wie beispielsweise mindestens 85 %, wie beispielsweise mindestens 90 %, wie beispielsweise mindestens 92 %, wie durch das Kjerdahl-Verfahren (Nx 6,25 %) an getrockneten Proteinproben bestimmt, zu erhalten.

9. Verfahren nach einem der vorhergehenden Ansprüche, wobei ein Rohmaterial für die wässrige Proteinlösung, die Protein und Chlorophyll umfasst, aus der Gruppe ausgewählt wird bestehend aus Pflanzenblättern, Stängeln und Hülsen; Cyanobakterien, Algen und Wasserpflanzen.

10. Verfahren nach Anspruch 9, wobei die Pflanzenblätter, Hülsen und Stängel von landwirtschaftlichen Nutzpflanzen wie beispielsweise Gräsern, Alfalfa, Kartoffel, Süßkartoffel, Spinat, Sorghum, Kassava, Reis, Zuckerrüben, Zuckerrohr, Bohnen und Erbsen stammen.

11. Verfahren nach Anspruch 9, wobei die Cyanobakterien aus einer *Spirulina-Spezies,* wie beispielsweise *Arthrospira platensis* und/oder *Arthrospira maxima* ausgewählt werden.

12. Verfahren nach Anspruch 9, wobei die Wasserpflanze von der Lemna-Gattung stammt, wie beispielsweise Falscher Buchweizen ist.

13. Chlorophyll-Silicat-Produkt, das nach einem der Ansprüche 1-12 hergestellt wird, bevorzugt 10-99 %, wie beispielsweise 15-95 %, 20-90 %, 30-90 %, 35-90 %, 40-90 % Chlorophyll und 1-90 %, wie beispielsweise 5-85 %, wie beispielsweise 10-80 %, wie beispielsweise 10-70, wie beispielsweise 10-65 %, wie beispielsweise 10-60 % Silicat, auf Trockengewichtsbasis, umfassend.

14. Verwendung eines Chlorophyll-Silicat-Produkts nach Anspruch 13 als ein Rohmaterial oder einen Bestandteil eines Nahrungsmittels, eines Futters, eines Kosmetikums, eines Nahrungsergänzungsmittels oder eines Gesundheitsfürsorgeprodukts.

15. Verwendung eines Chlorophyll-Silicat-Produkts nach Anspruch 13 als ein Rohmaterial oder einen Bestandteil für ein Sattheits- und/oder Gewichtskontrollprodukt.

## Revendications

1. Procédé de préparation de protéines solubles à partir de chlorophylle dans une solution aqueuse de protéine comprenant ladite protéine et de la chlorophylle, le procédé consistant à ;
a. fournir une solution aqueuse contenant une protéine soluble et de la chlorophylle
b. ajouter un silicate soluble dans l'eau à la solution de l'étape a) de telle sorte que la concentration totale de silicium sous la forme de silicates libres ou complexés dans la solution se situe dans la plage allant de 1 à 500 mM, telle que dans la plage allant de 2 à 300 mM, telle que dans la plage allant de 3 à 200 mM, telle que dans la plage allant de 3 à 100 mM, telle que dans la plage allant de 3 à 30 mM, telle que dans la plage allant de 3 à 20 mM, telle que dans la plage allant de 3,5 à 100 mM, telle que dans la plage allant de 4 à 60 mM, telle que dans la plage allant de 4 à 50 mM, telle que dans la plage allant de 5 à 30 mM
c. le cas échéant, ajuster le pH de la solution résultante à un pH dans la plage allant de pH 5 à pH 11, tel qu'un pH dans la plage allant de pH 5,5 à pH 10, tel qu'un pH dans la plage allant de pH 6,0 à pH 9,5, tel qu'un pH dans la plage allant de pH 6,2 à pH 9,0, tel qu'un pH dans la plage allant de pH 6,5 à pH 8,5, tel qu'un pH dans la plage allant de pH 6,0 à pH 7,5
d. permettre au silicate de former un précipité insoluble comprenant des complexes de silicate-chlorophylle, tandis que la protéine soluble reste soluble dans la solution
e. séparer les complexes de silicate-chlorophylle de la solution de protéine sous forme de précipité humide ; sous la forme d'un gâteau humide ou d'une suspension aqueuse du précipité,
f. éventuellement laver les complexes de silicate-chlorophylle,
g. éventuellement séparer la chlorophylle du silicate,
h. éventuellement isoler la protéine de la solution de protéine obtenue lors de l'étape e),
permettant ainsi d'obtenir la protéine et la chlorophylle dans des fractions séparées.

2. Procédé selon la revendication 1, dans lequel ladite étape f) de lavage, ladite étape g) de séparation et/ou ladite étape h) d'isolement est obligatoire.

3. Procédé selon l'une quelconque des revendications 1 ou 2 comprenant en outre une étape de filtration permettant d'éliminer des particules ou des fibres insolubles avant l'étape b).

4. Procédé selon l'une quelconque des revendications 1 à 3, dans lequel la température de ladite étape d) se situe dans la plage allant de 5 à 55 °C, telle que dans la plage allant de 7 à 50 °C, telle que dans la plage allant de 10 à 48 °C, telle que dans la plage allant de 15 à 45 °C, telle que dans la plage allant de 15 à 40 °C, telle que dans la plage allant de 10 à 30 °C.

5. Procédé selon l'une quelconque des revendications 1 à 4, dans lequel le précipité insoluble de l'étape d) contient moins de 50 %, tel que moins de 40 %, tel que moins de 30 %, tel que moins de 25 %, tel que moins de 20 %, tel que moins de 15 %, tel que moins de 10 % de ladite protéine soluble.

6. Procédé selon l'une quelconque des revendications 1 à 5, dans lequel les complexes de chlorophylle-silicate séparés dans ladite étape e) sont extraits avec un ou plusieurs parmi des solvants organiques, un acide, une base, des détergents ou des solutions aqueuses à haute force ionique ou des combinaisons de ceux-ci pour en séparer un ou plusieurs parmi les phénols, les pigments, les phytates, les saponines, les tanins ou les inhibiteurs de la protéase.

7. Procédé selon l'une quelconque des revendications précédentes, dans lequel la protéine provenant de la solution de protéine obtenue dans l'étape e) est isolée selon un procédé comprenant en outre un traitement de la solution de protéine en utilisant une filtration par membrane à flux tangentiel, dans lequel la protéine est retenue dans le rétentat et les impuretés passent à travers la membrane en tant que perméat, ou dans lequel la protéine provenant de la solution de protéine obtenue dans l'étape e) est isolée selon un procédé comprenant l'acidification de la solution de protéine pour former un précipité insoluble de la protéine et l'isolement du précipité, ou dans lequel la protéine provenant de la solution de protéine obtenue dans l'étape e) est isolée selon un procédé comprenant en outre une addition de silicate à la solution de protéine et, si nécessaire l'ajustement du pH, pour former un précipité insoluble de complexes de protéine-silicate et l'isolement du précipité.

8. Procédé selon l'une quelconque des revendications précédentes, dans lequel la protéine provenant de la solution de protéine obtenue dans l'étape e) est isolée pour obtenir une pureté d'au moins 50 %, telle que d'au moins 60 %, telle que d'au moins 65 %, telle que d'au moins 70, telle que d'au moins 75 %, telle que d'au moins 80 %, telle que d'au moins 85 %, telle que d'au moins 90 %, telle que d'au moins 92 %, telle que déterminée par la méthode de Kjeldahl (Nx 6,25 %) sur des échantillons de protéines séchées.

9. Procédé selon l'une quelconque des revendications précédentes, dans lequel une matière première pour la solution aqueuse de protéine comprenant une protéine et de la chlorophylle est choisie dans le groupe constitué par les feuilles, les gousses et les tiges de la plante ; les cyanobactéries, les algues et les plantes aquatiques.

10. Procédé selon la revendication 9, dans lequel les feuilles, les gousses et les tiges de la plante proviennent de cultures agricoles telles que les graminées, la luzerne, la patata, la patata douce, les épinards, le sorgho, le manioc, le riz, la betterave à sucre, la canne à sucre, le tabac, les haricots et les pois.

11. Procédé selon la revendication 9, dans lequel les cyanobactéries sont choisies à partir d'une espèce de spiruline, telle que *Arthrospira platensis* et/ou *Arthrospira maxima.*

12. Procédé selon la revendication 9, dans lequel la plante aquatique appartient au genre Lemna, telle que la lentille d'eau.

13. Produit de chlorophylle-silicate produit selon l'une quelconque des revendications 1 à 12, de préférence comprenant entre 10 et 99 %, tel que comprenant entre 15 et 95 %, tel que comprenant entre 20 et 90 %, tel que comprenant entre 30 et 90 %, tel que comprenant entre 35 et 90 %, tel que comprenant entre 40 et 90 % de chlorophylle, et comprenant entre 1 et 90 %, tel que comprenant entre 5 et 85 %, tel que comprenant entre 10 et 80 %, tel que comprenant entre 10 et 70 %, tel que comprenant entre 10 et 65 %, tel que comprenant entre 10 et 60 % de silicate, sur une base de poids sec.

14. Utilisation d'un produit de chlorophylle-silicate selon la revendication 13 en tant que matière première ou ingrédient pour une denrée alimentaire, un aliment pour animaux, un cosmétique, un complément alimentaire ou un produit de soins de santé.

15. Utilisation d'un produit de chlorophylle-silicate selon la revendication 13 en tant que matière première ou ingrédient pour un produit de contrôle de la satiété et/ou du poids.
